# EUROPEAN PATENT APPLICATION

(11) **EP 1 953 220 A1**
(43) Date of publication of application: **06.08.2008**
(21) Application number: 07290132.5
(22) Date of filing: 01.02.2007
(51) Int. Cl.: C12M 3/00

(54) **Method for applying mechanical loading to cells and apparatus for implementing such method**

(71) Applicant: INSERM (Institut National de la Santé et de la Recherche Médicale), 75654 Paris Cedex 13 (FR)
(72) Inventor: The designation of the inventor has not yet been filed
(74) Representative: Cabinet Plasseraud

(57) **Abstract**

A method for applying a mechanical loading to cells, comprising:
- a step of providing a support (1) having a bearing surface (2) adapted to receive said cells,
- a step of providing cells on the bearing surface (2) of said support (1),
- a step of placing said support (1) provided with the cells within a chamber (4) adapted to house said support (1),
- at least one step of applying a mechanical loading to the support (1) by a loading device (5) which extends at least in part in the chamber (4), a dynamic loading in vibration being applied to the support (1), said dynamic loading having a magnitude and a frequency and being adapted to cause the support (1) to vibrate.

## Description

The invention relates to a method for applying a mechanical loading to cells.

Mechanical loading to which cells composing for example a biological tissue or a microorganism are subjected is known to induce a response of the cells. Control of the mechanical loading applied to cells and definition of the appropriate mechanical loading to apply appear to be essential features in tissue formation as well as in microorganism development.

In particular, in the biomedical field, response of adherent cells, for example osteoblasts of a bone tissue, has been studied.

Actually, during human activities such as walking or running bone tissue is subjected to mechanical loading which is transmitted to the osteoblasts to make bone synthesis.

In the case of bone tissue, mechanical loading is particularly critical since it has been shown that the focal adhesion contacts, i.e. the structures linking the cytoskeleton of the osteoblasts to an extracellular matrix, are reorganised with regard to the mechanical loading, said reorganisation being a reinforcement in case of increase of the mechanical loading and said reorganisation being a dismantling in case of decrease of the mechanical loading.

Besides, if a lack of mechanical loading applied to osteoblasts leads to deterioration or weakening of the bone tissue, an excess of mechanical loading might lead to wrong excessive bone tissue reinforcement.

Thus, external mechanical loading can be transduced into biological signals to adapt osteoblast internal activities and the balance between external loading and internal activities is a fundamental element for controlling the bone tissue mechanical properties which allow the bone tissue to play its role.

Furthermore, in order to repair a tissue default and to overcome the limits of auto-graft in terms of pain, availability, infection risks, it is now turned to the making of artificial tissues, prosthesis or implants where adherent cells, for example osteoblasts, are cultured on an appropriate support, for example a substrate made of biomaterial. In such making, several aspects have to be taken into account such as the type of cell, the type of biomaterial and the culture conditions. Another important aspect to be considered is the modulation of cell growth and synthesis by mechanical loading.

Actually, whereas mechanical loading plays an essential role for cell synthesis and adhesion, mechanical loading can make implantation fail or render artificial tissues, prosthesis or implants inefficient if cells at the interface between the support and the cells are not able to stabilize said interface by the deposition of a mineralized extracellular matrix.

*In vivo* studies have been performed to measure the mechanical deformations in bone tissue during walking and to determine strain regimens that are more efficient to stimulate bone tissue formation.

Further, in order to be able to perform *in vitro* studies, methods for applying a mechanical loading to cells in an *in vitro* way have been implemented. Said methods comprise:
- a step of providing a support having a bearing surface adapted to receive said cells,
- a step of providing cells on the bearing surface of said support,
- a step of placing said support provided with the cells within a chamber adapted to house said support,
- at least one step of applying a mechanical loading to the support by a loading device which extends at least in part in the chamber.

In known methods, the mechanical loading is applied to the support in the form of strains by a bending, a tension or a compression in order to obtain a deformation of the support.

However, in such methods, the mechanical loading is applied locally and do not provide for a mechanical loading that is uniform on the whole support. Thus, said mechanical loading cannot be controlled in a satisfactory way.

Furthermore, since cells on the support cannot be submitted to the same mechanical loading, known methods do not give satisfaction in terms of liability and reproducibility.

Besides, the methods are difficult to adapt to different types of support, especially said methods are not appropriate for rigid supports.

The invention aims to solve the above mentioned deficiency.

To this end, the invention concerns a method of the aforementioned type wherein, at the step of applying the mechanical loading, a dynamic loading in vibration is applied to the support, said dynamic loading having a magnitude and a frequency and being adapted to cause the support to vibrate.

Hence, the dynamical loading, of which magnitude and frequency can be easily controlled, causes the whole support to vibrate and to oscillate. Vibrations of the support operate uniformly on the cells, so that the method offers the desired liability and reproducibility. The method can be implemented with any type of support.

Further, such method stimulates cell activity in reinforcing interactions between the cells and at the interface between the cells and the support by taking into account the dynamic factors, especially the dynamic behaviour of the support, which occur by real or in vivo conditions.

In a particular embodiment, the step of applying the dynamic loading may comprise applying an impact. Applying an impact, i.e. a shock, a loading suddenly applied, on the support eases the control of the mechanical loading and insures that the support will vibrate in a controlled manner.

The step of applying the dynamic loading may comprise varying said magnitude in a range of magnitudes and/or said frequency in a range of frequencies. This feature improves control of the dynamic loading and allows adaptation of the dynamical loading according to the type of cells and/or to the type of support.

Besides, the dynamic loading may be applied perpendicularly and/or parallel to the bearing surface.

The dynamic loading may be applied to a surface that represents more than 50% of a plane face of the support which faces the loading device so that an evenly distributed loading can be applied to the support.

In an embodiment, the method may be implemented for cell culture under dynamic loading.

The method may further comprise, after the step of providing cells on the bearing surface of said support, placing the support in a culture environment. The chamber may be adapted to be closed in a tight way, the culture environment may comprise a fluid culture medium, said placing the support in the culture environment comprising, after the step of placing said support within the chamber, closing the chamber in a tight way and supplying the chamber with said fluid culture medium. Said placing the support in the culture environment may also comprise placing the support in an incubator.

Furthermore, the cells may be adherent cells, the support may be a substrate made of a biomaterial. Such method may be implemented for making an implant. Hence, the method may be used to give a specific function to the implant by choosing the appropriate cells and substrate.

The implant, in which cells have been cultured so as to improve adhesion to the support by producing an efficient extracellular matrix at the interface with the support, offers an enhanced biocompatibility and an improved mechanical efficiency.

In an embodiment, the method may further comprise a step of determining dynamic properties of the support by a dynamic data collection device.

The method allows characterising the support with its dynamic properties so as to be able to identify the appropriate support for cell genesis and extracellular matrix interfacing of the cells on the support. Besides, the shape and/or the material of the support could be chosen according to the aiming function of the cells or of the assembly of the cell and the support. For example, when making an implant or a cell culture in the biomedical field, the support could be chosen to enhance cell genesis, biocompatibility or to improve mechanical efficiency.

A dynamic analysis of the support allows acknowledgement of the support dynamic behaviour which will more or less filter vibrations generated by the dynamic loading and effectively received by the cells.

Such method permits to measure the mechanical loading transmitted to and received by the cells rather than to the mechanical loading applied to the support. Thus, transduction mechanism of the dynamical loading into biological signals which govern cell activities may be studied and better understood so as to be able to correlate the type of support with an expected cell response according to the dynamic loading.

In particular, the step of determining the dynamic properties of the support may comprise measuring, recording and processing force applied to the support and/or acceleration of the support and, eventually, performing a vibratory analysis of the support.

The step of applying the dynamic loading may comprise controlling the magnitude and/or the frequency of dynamic loading according to the determined dynamic properties of the support. Thus, said method allows establishment of a correlation between the dynamical properties of the support and the dynamic loading.

The method may further comprise, after the step of applying the dynamic loading, a step of analysing cell response to dynamic loading. Thus, cell culture conditions, especially in terms of dynamical loading, which enhance extracellular matrix interfacing, can be defined for example with regard to the type of cells or to the type of support. A correlation between cell response and dynamic loading can be established, another correlation between cell response and dynamic properties of the support being then possible.

According to another aspect, the invention concerns an apparatus for implementing the above defined method, comprising:
- a frame,
- a chamber mounted on the frame, adapted to house a support, said support having a bearing surface adapted to receive cells,
- a loading device mounted on the frame, extending at least in part in the chamber and adapted to apply a mechanical loading to the support,
the loading device being adapted to apply to the support a dynamic loading in vibration, said dynamic loading having a magnitude and a frequency and being adapted to cause the support to vibrate.

The loading device may be adapted to apply an impact.

The loading device may be adapted to vary said magnitude in a range of magnitudes and/or said frequency in a range of frequencies.

The loading device may be adapted to apply the dynamic loading perpendicularly and/or parallel to the bearing surface.

The support may have a plane face which faces the loading device and the loading device may be adapted to apply the dynamic loading to a surface that represents more than 50% of said face.

The chamber may be adapted to be closed in a tight way and may be provided with a supply device for a fluid culture medium.

In an embodiment, the apparatus may further comprise a dynamic data collection device adapted to determine dynamic properties of the support.

In particular, the dynamic data collection device may comprise at least an acceleration sensor adapted to output a signal representing acceleration of the support, and a central unit, which acceleration sensor is connected to, said acceleration sensor being adapted to measure the acceleration of the support, said central unit being adapted to record and process the signal output by the acceleration sensor and, eventually, to perform a vibratory analysis of the support. Besides, the dynamic data collection device may further comprise at least a force sensor adapted to output a signal representing force applied to the support, said force sensor being connected to the central unit and being adapted to measure the force applied to the support, said central unit being adapted to record and process the signal output by the force sensor.

Other objects and advantages of the invention will emerge from the following disclosure made in reference to the enclosed drawings in which:
- Figure 1 is a perspective view of an apparatus for applying a mechanical loading to cells according to a first embodiment of the invention,
- Figure 2 is a sectional view of the apparatus of Figure 1,
- Figure 3 is an enlarged view of the detail referenced III on Figure 2 and representing a chamber of the apparatus,
- Figure 4 is a view of the chamber of Figure 3 in an instrumented state,
- Figure 5 is part view of the detail referenced V on Figure 3,
- Figure 6 is an enlarged view of the detail referenced VI on Figure V,
- Figure 7 is part sectional perspective view of an apparatus for applying a mechanical loading to cells according to a second embodiment of the invention,
- Figure 8 is a sectional view of the apparatus of Figure 7,
- Figure 9 is an enlarged view of the detail referenced IX on Figure 8,
- Figure 10 is a perspective view of a case of the apparatus of Figure 7,
- Figure 11 is a perspective view of a holder of the apparatus of Figure 7,
- Figure 12 is a graph representing acceleration with regard to the time of a support submitted to mechanical loading by the apparatus of Figure 1,
- Figure 13 is a graph representing force with regard to the time applied to a support submitted to mechanical loading by the apparatus of Figure 1,
- Figure 14 is a graph obtained by a frequency analysis of the acceleration of Figure 12, representing the frequency response of the support to the mechanical loading,
- Figure 15a is a diagram representing the number of stress fibers per cell four hours after mechanical shocks have been applied at 1 hit per second (Hps) and at 10 Hps to a support provided with cells compared to the number of stress fibers per cell for unsolicited cells,
- Figures 15b, 15c and 15d represent F-actin polymerisation four hours after mechanical shocks, respectively for unsolicited cells, for cells stimulated at 1 Hps and for cells stimulated at 10 Hps,
- Figure 15e is a diagram representing the number of stress fibers per cell twenty-four hours after mechanical shocks have been applied at 1 Hps and at 10 Hps to a support provided with cells compared to the number of stress fibers per cell for unsolicited cells,
- Figures 15f, 15g and 15h represent F-actin polymerisation twenty-four hours after mechanical shocks, respectively for unsolicited cells, for cells stimulated at 1 Hps and for cells stimulated at 10 Hps,
- Figure 16a is a diagram representing the mean vinculin area four hours after mechanical shocks have been applied at 1 Hps and at 10 Hps to a support provided with cells compared to the mean vinculin area for unsolicited cells,
- Figures 16b, 16c and 16d represent vinculin staining four hours after mechanical shocks, respectively for unsolicited cells, for cells stimulated at 1 Hps and for cells stimulated at 10 Hps,
- Figure 16e is a diagram representing the mean vinculin area twenty-four hours after mechanical shocks have been applied at 1 Hps and at 10 Hps to a support provided with cells compared to the mean vinculin area for unsolicited cells,
- Figures 16f, 16g and 16h represent vinculin staining twenty-four hours after mechanical shocks, respectively for unsolicited cells, for cells stimulated at 1 Hps and for cells stimulated at 10 Hps,
- Figure 17a is a diagram representing the mean FAK-397P area twenty-four hours after mechanical shocks have been applied at 1 Hps and at 10 Hps to a support provided with cells compared to the mean FAK-397P area for unsolicited cells,
- Figures 17b, 17c and 17d represent FAK-397P staining twenty-four hours after mechanical shocks, respectively for unsolicited cells, for cells stimulated at 1 Hps and for cells stimulated at 10 Hps,
- Figure 18a is a diagram representing the fibronectine area normalized by the DAPI area forty-eight hours after mechanical shocks have been applied at 1 Hps and at 10 Hps to a support provided with cells compared to the fibronectine area normalized by the DAPI area for unsolicited cells,
- Figures 18b, 18c and 18d represent fibronectine staining with its respective DAPI staining forty-eight hours after mechanical shocks, respectively for unsolicited cells, for cells stimulated at 1 Hps and for cells stimulated at 10 Hps,
- Figure 19a is a diagram representing the soluble collagen (Coll-FITC) area normalized by the DAPI area forty-eight hours after mechanical shocks have been applied at 1 Hps and at 10 Hps to a support provided with cells compared to the Coll-FITC area normalized by the DAPI area for unsolicited cells,
- Figures 19b, 19c and 19d represent Coll-FITC staining with its respective DAPI staining forty-eight hours after mechanical shocks, respectively for unsolicited cells, for cells stimulated at 1 Hps and for cells stimulated at 10 Hps,
- Figure 20a is a diagram representing the average BrdU positive cells forty-eight hours after mechanical shocks have been applied at 1 Hps and at 10 Hps to a support provided with cells compared to the average BrdU positive cells for unsolicited cells,
- Figures 20b, 20c and 20d represent BrdU staining with its respective DAPI staining forty-eight hours after mechanical shocks, respectively for unsolicited cells, for cells stimulated at 1 Hps and for cells stimulated at 10 Hps,
- Figure 21a is a diagram representing the average TUNEL positive cells forty-eight hours after mechanical shocks have been applied at 1 Hps and at 10 Hps to a support provided with cells compared to the average TUNEL positive cells for unsolicited cells,
- Figures 21b, 21c and 21d represent TUNEL staining with its respective DAPI staining forty-eight hours after mechanical shocks, respectively for unsolicited cells, for cells stimulated at 1 Hps and for cells stimulated at 10 Hps.

On the Figures, same references refer to similar or analogous elements.

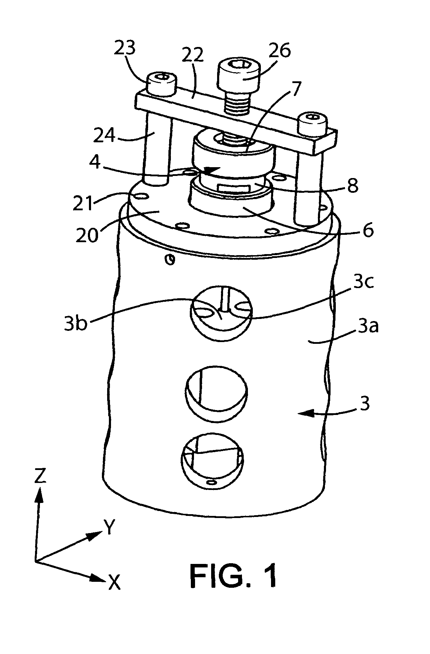

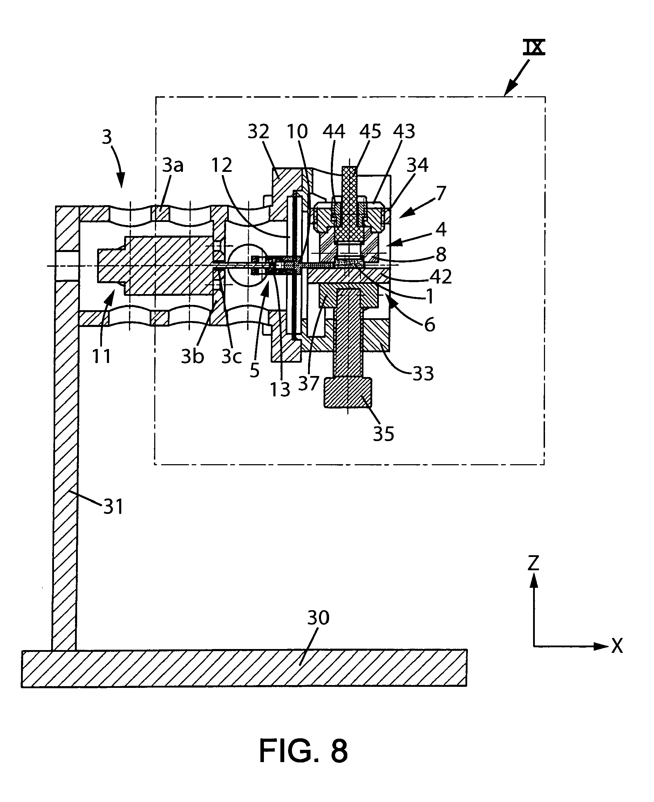

On Figures 1 to 11, an apparatus for applying a mechanical loading to cells is represented.

Said apparatus is used to apply mechanical loading, in a controlled, uniform, reproducible and liable manner, to cells resting on a support 1. Such apparatus may be used in many fields in order, for example, to culture cells on an appropriate support 1 while enabling to study and to control the effects of mechanical loading on cell response and on cell activity, and/or the mechanism of transduction.

Cells may be of any desired type, for example adherent cells, stem cells, and said cells may belong to the animal kingdom or to any other kingdom. Non limitative examples of cells may be osteoblastic cells, mesenchymatous stromal cells, hematopoietic cells, endothelial cells, cartilaginous cells, odontoblastic cells, fungous cells, bacteria, microorganism or other.

Cells are provided on the support 1 which may be of any suitable shape or material. Said support 1 has a bearing surface 2 adapted to receive cells. The support may be rigid or flexible, artificial or natural, porous or not and, for example, made of ceramic, metal, polymer, composite, bone or other.

For example, on the Figures, the support 1 may be in the form of a flat pellet having opposite plane upper 1a and lower 1b faces and a peripheral outer edge 1c between said upper 1a and lower 1b faces. The upper face 1a of the support 1 may have the bearing surface 2. The outer edge 1c may be substantially circular, elliptic, polygonal or other and extends substantially perpendicularly to the upper 1a and lower 1b faces. However, in case of need, the support 1 may be thicker.

In a particular embodiment which will be disclosed later together with an illustrative example of implementation, the apparatus may be used in the biomedical field to culture cells on a support 1 under mechanical loading, to make an implant, prosthesis or an artificial tissue or to study cell response according to the mechanical loading and/or to the support 1.

Cells may then be adherent and the support 1 may be a substrate made of a biomaterial. A biomaterial or biocompatible material is an artificial or a natural material which is tolerated by a living organism and which, once implanted in the living organism, do not cause any rejection or toxic reaction, lesion or other negative effect on said organism.

The apparatus comprises:
- a frame,
- a chamber 4 mounted on the frame and adapted to house the support 1, and
- a loading device 5 mounted on the frame, extending in the chamber 4 and adapted to apply a mechanical loading to the support 1.

The frame may comprise a casing 3 made of a rigid material and comprising a hollow cylindrical wall 3a. Said hollow cylindrical wall 3a defines an inner space and has a first open end and a second open end which is opposite the first end.

On the Figures, the casing 3 may also comprise a transverse intermediate wall 3b between the two ends of the cylindrical wall 3a, said intermediate wall 3b having a central orifice 3c and dividing the inner space into two compartments.

The chamber 4 may be mounted on the second end of the cylindrical wall 3a.

The chamber 4 may be delimited by a bottom 6 and a hollow cylindrical body 8. In case of need, the chamber may also be delimited by a lid 7 opposite the bottom 6 so as to be closed. The bottom 6 and the lid 7 may be fixed to the body 8, at least one of the lid 7 and the bottom 6 being fixed to the body 8 in a removable manner. Besides, the bottom 6 and the lid 7 may be fixed to the body 8 in a tight manner.

The chamber 4 may be adapted to hold the support 1 by any suitable holding means so that the support 1 extends in plane substantially horizontal, the holding means being added or integrated to the chamber 4.

For example, on the Figures, the support 1 may be held along a peripheral area between an end of the body 8 and the bottom 6. At least one of the lid 7 and the bottom 6 may then be fixed to the body 8 in an adjustable manner so as to control strains applied to the support 1 for holding it. To provide tightness, one or several seal joints 9 may be used in appropriate locations.

To be able to apply controlled mechanical loading close to the real or in vivo conditions, the loading device 5 is adapted to apply a dynamic loading in vibration to the support 1. The dynamic loading has a magnitude and a frequency, either or both of which can be varied in a range of magnitudes and in a range of frequencies, respectively, by the loading device 5.

A dynamic loading in vibration can be understood as any loading able to cause a vibratory movement, oscillatory movement of the support. The dynamic loading may change very rapidly, for example, in terms of direction, magnitude and/or frequency.

For example, the dynamic loading may be an impact, a shock or any suddenly applied loading.

In the figures, the loading device 5 may be adapted to apply an impact or a series of impacts to the support in one direction. However, impacts could be imparted alternatively in two opposite directions or in any suitable manner so as to cause the support 1 to vibrate.

The loading device 5 may comprise a hitting member 10, for example a titanium hammer, extending through an opening of the chamber 4 to be able to apply directly the dynamic loading to the support 1. However, the loading device 5 might be of any other convenient type, and may extend totally in the chamber 4.

For example, the loading device 5 may further comprise an electromagnetic actuator 11 adapted to drive the hitting member 10 in a reciprocating movement between a hitting position, where said hitting member 10 hits the support 1, and a distant position, where said hitting member 10 is apart from the support 1. The electromagnetic actuator 11 may comprise, in a known manner, a solenoid 11a and a stalk 11b on which the hitting member 10 is mounted.

The loading device 5 may be mounted in the inner space of the casing 3. In particular, the solenoid 11a may be arranged in the compartment close to the first end of the cylindrical wall 3a, the stalk 11b extending through the orifice 3c of the intermediate wall 3b towards the second end and the chamber 4.

To position the hitting member 10 and to guide the reciprocating movement of said hitting member 10, the loading device 5 may comprise a guiding member 12, in the form, for example, of a deformable membrane or of a series of spokes which extends between the cylindrical wall 3a of the casing 3 and the hitting member 10. Furthermore, the loading device 5 may have a linking element 13 adapted to connect the hitting member 10 on one side of the guiding member 12 to the stalk 11b of the electromagnetic actuator 11 on the other side of the guiding member 12. The hitting member 10 may have a threaded stem adapted to be screwed in a threaded orifice of the linking element 13, the guiding member 12 having a central hole through which the stem of the hitting member 10 extends, the edge of the central hole being held between the guiding member 12 and the hitting member 10.

Different arrangements of the casing 3, the chamber 4 and the loading device 5 may be provided. Two exemplary embodiments of arrangement will now be described.

According to a first embodiment represented on Figures 1 to 6, the loading device 5 is adapted to apply the impact perpendicularly to the bearing surface 2 of the support 1, and in particular, to apply the impact to the lower face 1b of the support 1 opposite the bearing surface 2.

The casing 3 may rests on the first end of the cylindrical wall 3a so as to extend along a vertical direction z.

The frame may comprise a mounting plate 20 fixed to the second end of the cylindrical wall 3a and provided with threaded through holes 21 at its periphery. A traverse 22 may be mounted on the mounting plate 20 opposite the cylindrical wall 3a, at a distance from the mounting plate 20, by means of bolts 23 extending through spacer sleeves 24 to the through holes 21 of the mounting plate 20. The outer periphery of the guiding member 12 may be placed between the mounting plate 20 and an annular groove formed in the second end of the casing 3. Eventually, a ring 25 may be interposed between the guiding member 12 and the casing 3.

The chamber 4 may be mounted between the mounting plate 20 and the traverse 22. The bottom 6 of the chamber 4 may be disposed in a central recess formed in the mounting plate 20. Further, to secure the chamber 4 to the frame, a tightening bolt 26 may by connected to the traverse so as to urge the lid 7 of the chamber 7.

In this embodiment, the lid 7 and the bottom 6 of the chamber 4 may have internal thread and the body 8 may have an external thread.

The lid 7 may comprise a ring 7a comprising said thread and substantially surrounding a plug 7b. The plug 7b is partly inserted in the body 8 and has a peripheral shoulder resting on the upper end 8a of the body 8. The tightening bolt 26 is placed in contact with the plug 7b.

The bottom 6 has a central opening 6a though which the hitting member 10 extends to apply the impact to the lower face 1b of the support 1.

The opening 6a may be closed by the support 1. And tightness may be provided by the seal joint 9 disposed between the lower end 8b of the body 8, the outer edge 1c of the support 1 and the bottom 6. The lower end 8b of the body 8 may be tapered widening towards the outside, with an angle for example of 45°, so that the seal joint 9 is pressed against the outer edge 1c of the support 1 as the body 8 is forced toward the bottom 6 by screwing the bottom 6 or the lid 7 on the body 8.

However, in case of a porous support 1, tightness might be provided by any appropriate means such as a flexible membrane covering the opening 6a, a lip in seal contact with the hitting member 10 or other.

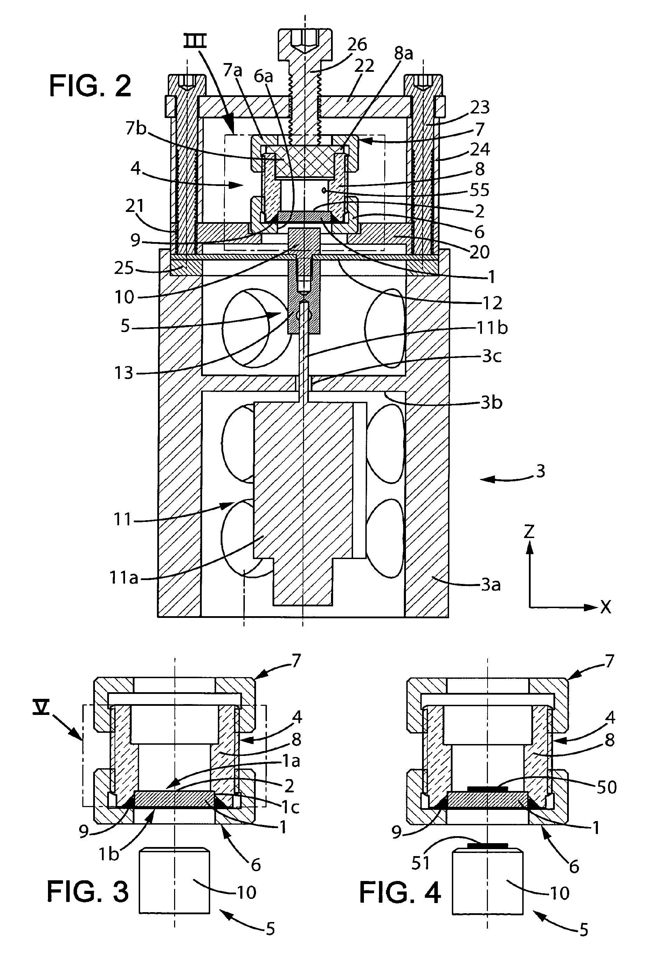

As best shown on Figures 3 and 4, the hitting member 10 has a contact area adapted to hit the lower face 1b of the support 1 in the hitting position, said contact area representing more than 50%, in particular more than 75%, and especially about 90%, of the lower face 1b of the support 1. Then the dynamic loading may be applied to a surface of the support 1 that is more than 50%, in particular more than 75%, and especially about 90%, of the lower face 1b of the support 1 so as to improve uniformity of the application of the loading.

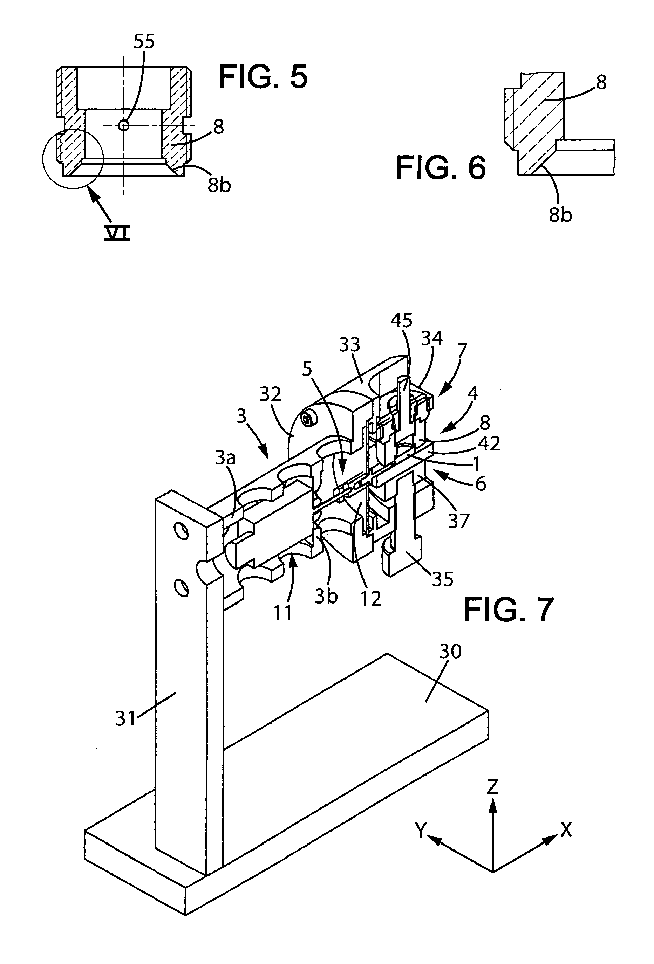

In a second embodiment represented on figures 7 to 11, the loading device is adapted to apply the impact parallel to the bearing surface 2 of the support 1, and in particular, to apply the impact to the outer edge 1c of the support 1.

The casing 3 may extend in a longitudinal direction X substantially horizontal. To that end, the frame may further comprise a base 30 adapted to rest on a flat surface and a fixing arm 31 extending in the vertical direction Z from the base 30. The fixing arm 31 has a free end, the first end of the cylindrical wall 3a of the casing 3 being connected thereto.

The second end of the casing 3 may have an outer flange 32, a case 33 being fixed thereto. In this embodiment, the outer periphery of the guiding member 12 may be held between said outer flange 32 and said case 33.

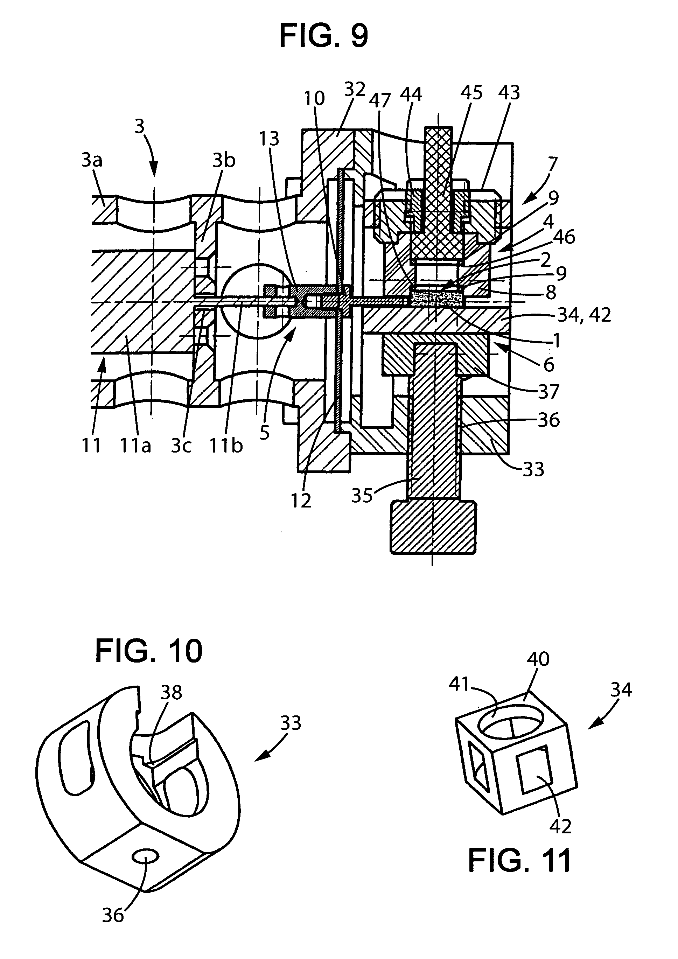

The case 33, best shown on Figure 10, may define a housing adapted to receive a holder 34, represented on Figure 11, which in turn receives the body 8 of the chamber 4. In particular, a tightening bolt 35 extending through an opening 36 of the case 33 may urge the holder 34, by means of a block 37, towards a shoulder 38 provided in the housing of the case 33 opposite the opening 36.

The holder 34 may have a top wall 40 provided with an aperture 41 and a bottom wall 42 which forms the bottom 6 of the chamber 4.

The lid 7 may then be mounted in the aperture 41 of the holder 34. In the figures, the lid 7 may comprise a fixing ring 43 provided with an external thread which cooperates with an internal thread of the aperture 41. Said fixing ring 43 may surround a tightening screw 44 having a plug 45 extending there through. The plug 45 may then press against an inner annular shoulder 46 of the body 8, a seal joint 9 being for example interposed between said shoulder 46 and said plug 45.

The support 1 may be held between the lower end of the body 8 and the bottom wall 42 of the holder 34 while leaving a free space to enable passage of the hitting member 10. A seal joint 9 may be placed within an annular groove 47 formed on the lower end so as to come in tight contact with the support 1.

Other arrangements of the apparatus are possible, for example, an arrangement wherein the dynamical loading would be applied both parallel and perpendicularly to the bearing surface 2 can be made.

As it can be seen on figure 4, in relation with the first embodiment of the apparatus, the apparatus may comprise a dynamic data collection device adapted to determine dynamic properties of the support 1.

In particular, the dynamic data collection device may comprise at least one acceleration sensor 50 adapted to output a signal representing acceleration of the support 1 and a central unit, non represented, which acceleration sensor 50 is connected to.

The acceleration sensor 50, in the form for example of an accelerometer fixed to the upper face 1a of the support 1, may being adapted to measure the acceleration of the support 1. It may be provided that the central unit records and processes the signal output by the accelerometer 50 and performs a vibratory analysis of the support 1.

The dynamic data collection device may further comprise at least one force sensor 51 placed for example on the contact area of the hitting member 10 and adapted to output a signal representing force applied to the support. The force sensor 51 is connected to the central unit so as to measure the force applied to the support 1 by the hitting member 10, the central unit recording and processing the signal output by the force sensor 51.

The dynamic collection device can be used in any embodiment of the apparatus and may be of any convenient type, for example, the use of a contactless sensor, such as optical, laser sensor instead of a contact sensor such as the accelerometer 50 may be provided.

Thus, the frequency signature of the support 1 and the force applied to the support 1 can be obtained and used in order to determine the loading transmitted by the support 1 and received by cells. Determination of the appropriate loading to apply according to the dynamic response of the support 1, while taking into account the transduction mechanism, is then possible to study cell response according to the dynamic loading or to the type of support 1.

The loading, in particular in terms of magnitude and frequency, may be chosen to enhance cell activity in extracellular matrix interfacing formation to promote cell adhesion to the support and cell cohesion. In the making an implant, the controlling of the loading according to the type of support 1 permits to enhance biocompatibility, to functionalise the implant and to improve mechanical efficiency of the implant. On the contrary, dynamic loading regimen according to the support type that decreases cell adhesion to the support and cell cohesion can be searched.

To improve cell culture conditions, the chamber 4 of the apparatus may be provided with a supply device for a fluid culture medium. Orifices 55 may be formed in the chamber to allow supplying fluid culture medium by suitable conduits. The supply device may be adapted to renew or to change the fluid culture medium. The medium may be circulating so as to add shear strains to the support or to enable study of cell response by analysing, in particular chemically, the composition of the medium outflow. Then the supply device may be used to measure and to control the changing components or the changing state of the culture conditions and of the fluid culture medium, for example the pH, the composition or other. In a variant, the medium may be stagnant.

Furthermore, the apparatus may be used in a system comprising several apparatus and an incubator which can receive one or several apparatus in a culture environment, where for example temperature, moisture and gaseous composition of the atmosphere can be set.

The above described apparatus may be used to implement a method for applying a mechanical loading to cells. The method may be implemented for cell culture under dynamic loading.

In exemplary applications, the method may be implemented for culturing cells or making an implant, prosthesis or an artificial tissue. As stated above, the cells may then be adherent cells, the support 1 may be a substrate made of a biomaterial.

The method comprises:
- a step of providing the support 1 having the bearing surface 2 adapted to receive cells,
- a step of providing cells on the bearing surface 2 of said support 1,
- a step of placing said support 1 provided with the cells within the chamber 4 of the apparatus,
- at least one step of applying a dynamic loading in vibration to the support by the loading device 5 which extends at least in part in the chamber 4, the dynamic loading having a magnitude and a frequency and being adapted to cause the support to vibrate.

At each step of applying a dynamic loading, an impact or a shock, of which magnitude and/or frequency may vary in a range of magnitudes and/or in a range of frequencies, respectively, may be applied to the support 1.

As indicated above, the impact may be applied perpendicularly and/or parallel to the surface that represents more than 50%, in particular more than 75%, especially about 90%, of the lower face 1b of the support 1 which faces the loading device 5.

To control culture conditions, the method may further comprise, after the step of providing cells on the bearing surface 2 of said support 1, placing the support 1 in a culture environment.

This can be performed by :
- closing the chamber 4 in a tight way, the culture environment comprising the fluid culture medium,
- after the step of placing said support 1 within the chamber 4, closing the chamber in a tight way, and
- supplying the chamber 4 with the fluid culture medium.

In an eventually complementary variant, said placing the support in the culture environment may comprise placing the support 1, for example held in the chamber 4 with the lid 7 removed, in an incubator.

To make an artificial tissue, the method may further comprise a step of removing the cells with the extracellular matrix from the substrate to obtain an implantable efficient tissue.

Furthermore, to be able to correlate the dynamic loading regimen with cell response taking into account the transduction mechanism, the method may further comprise a step of determining dynamic properties of the support by the dynamic data collection device so that the loading effectively received by the cells can be determined.

The step of determining the dynamic properties of the substrate may be performed either before the step of providing cells to the support 1, by use of a contact sensor such as accelerometer 50 or other, or after the step of providing cells to the support 1, by use of a contactless sensor, optical, laser.

The method may comprise several steps of applying the dynamic loading. The step of determining dynamic properties of the support may be performed by connecting the dynamic data collection device at a single step of applying the dynamic loading, the dynamic data collection device being removed after said single step.

The step of determining the dynamic properties of the support may comprise measuring, recording and processing force applied to the support and/or acceleration of the support and performing a vibratory analysis of the support.

According to the vibratory analysis, to optimise cell response to the dynamical loading, the step of applying the dynamic loading may comprise controlling the magnitude and/or the frequency of dynamic loading according to the determined dynamic properties of the support.

The method may further comprise, after the step of applying the dynamic loading, a step of analysing cell response to dynamic loading, for example by imaging the cells, a chemical, statistical or visual analysis of the support and the cells, a chemical, statistical or visual analysis of the medium outflow or any other suitable means. Examples of a cell response analysis will follow.

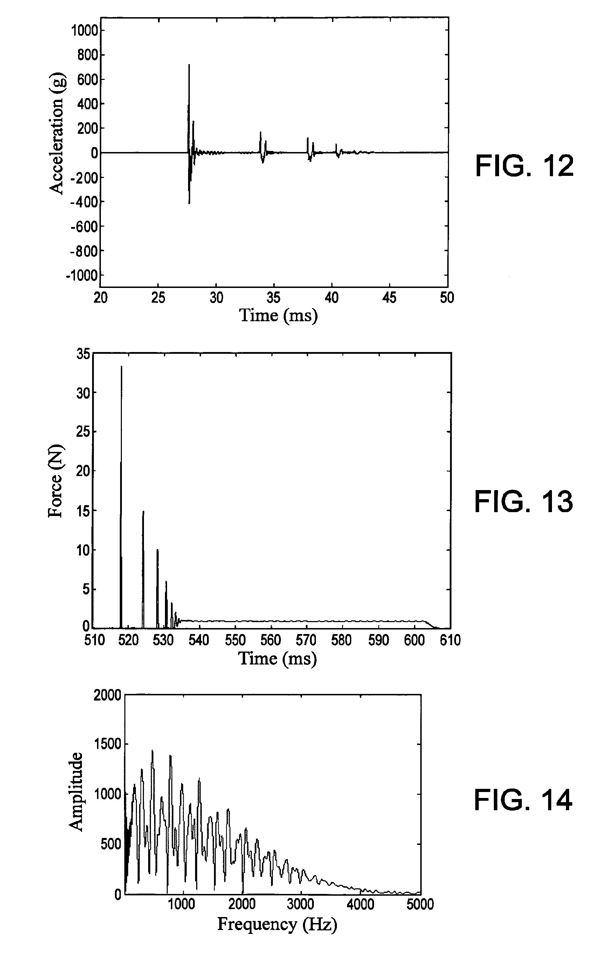

In relation with Figures 12 to 21, an illustrative example of implementation of the above described method with an apparatus according to the first embodiment will be described. In the example, the method is implemented to study the cell response of adherent cells, in particular osteoblasts, disposed on a support made of biomaterial submitted to impacts at two different frequencies.

The above described apparatus according to the first embodiment represented on Figures 1 to 6 has been used with supports 1 in the form of discs made of titanium alloy (Ti6Al4V), each disc being of 10 mm in diameter and 2 mm thick for Atomic Force Microscope compatibility. Each disc has been sealed into the chamber 4 filled with a culture medium. In this study, the mechanical shocks have been applied at two shocks frequencies: 1 hit per second (Hps) and 10 Hps.

To perform signal acquisition, two sensors, the accelerometer 50 and the force sensor 51, are coupled to the disc 1 and to the hitting member 10, respectively and connected to the central unit so that force signal (Figure 12) and acceleration signal (Figure 13) may be recorded with a sampling rate of 100 kHz. Force signal and acceleration signal may be recorded simultaneously during the impact and signal samples are interlaced to assure synchronized acquisition.

### Experimentation

### Titanium alloy disc preparation

Titanium alloy (Ti6Al4V) has a high biocompatibility and interesting mechanical properties. Mirror polished Ti6A14V discs (10 mm diameter, 2 mm thick, manufactured by ATI, Titanium International, France) are used. Before experiments, discs are sonicated using Milli-Q water, followed by immersion in ethanol for 10 min and sterilised at 180°C for 2 hours.

### Bone forming cells culture

MC3T3-E1 cells are plated onto Ti6Al4V discs at a density of 20.10³cells/cm² in alpha minimum Eagle's medium (α-MEM, Sigma) supplemented with 10% fetal calf serum (FCS, Promocell), 2 mM L-glutamine, 100 units/ml penicillin, and 0.1 mg/ml streptomycin. The medium is changed after the first forty-eight hours and replaced with a 2% FCS-containing medium prior mechanical stimulation. Chambers 4 and loading devices 5 are placed in an incubator (37°C, 95% humidity, 5% CO₂ in air) until the beginning of application of the shocks. The lid 7 is removed to allow gas exchanges during the incubation time, and replaced during mechanical experiments.

### Immunofluorescent staining

Four hours or twenty-four hours after application of the shocks, the cells are gently washed in Phosphate Buffered Saline (PBS) and fixed with Paraformaldhehyde 4% for 10 min at room temperature. Samples were washed with PBS and permeabilized with 0.1 % Triton x100 for four minutes at room temperature. Mouse monoclonal anti-vinculin antibody (HVIN-1, 1:100 dilution) is purchased from Sigma, France. Rabbit polyclonal phosphospecific antibody anti-FAK-Tyr(P)-397 (1:100 in 1% PBS/bovine serum albumin), is purchased from Biosource International (Camarillo, CA). Cells are incubated with fluorescein isothiocyanate-conjugated (FITC) goat anti-mouse or anti-rabbit IgG (1:100 in PBS/bovine serum albumin 1%, Sigma, France) for one hour at 37°C. Hoeschst stain (1:200, Sigma, France) is used to label the cells nuclei: samples were placed in incubator (37°C, 5% CO₂) for ten minutes. Cells were extensively washed in PBS between incubations and mounted in Fluoprep (BioMerieux, France) before microscopic examination.

### Image acquisition and analysis

Immunostaining is observed with Fluorescence microscope (Leica DMRB, Lyon, France) and acquired with a cooled CCD camera (CoolSNAP.fx, Roper Scientific, Evry, France) with Metaview acquisition software (Universal Imaging Corp, Downingtown, PA, USA). Images (650x515, 8bits) are analyzed using the SAMBA IPS-640 image cytometer (Alcatel-TITN, Grenoble, France). A specific application was written in the proprietary image oriented language of the SAMBA system. Averages of 100 cells or 20 fields per experimental group are analyzed (Usson, Guignandon et al. 1997) .

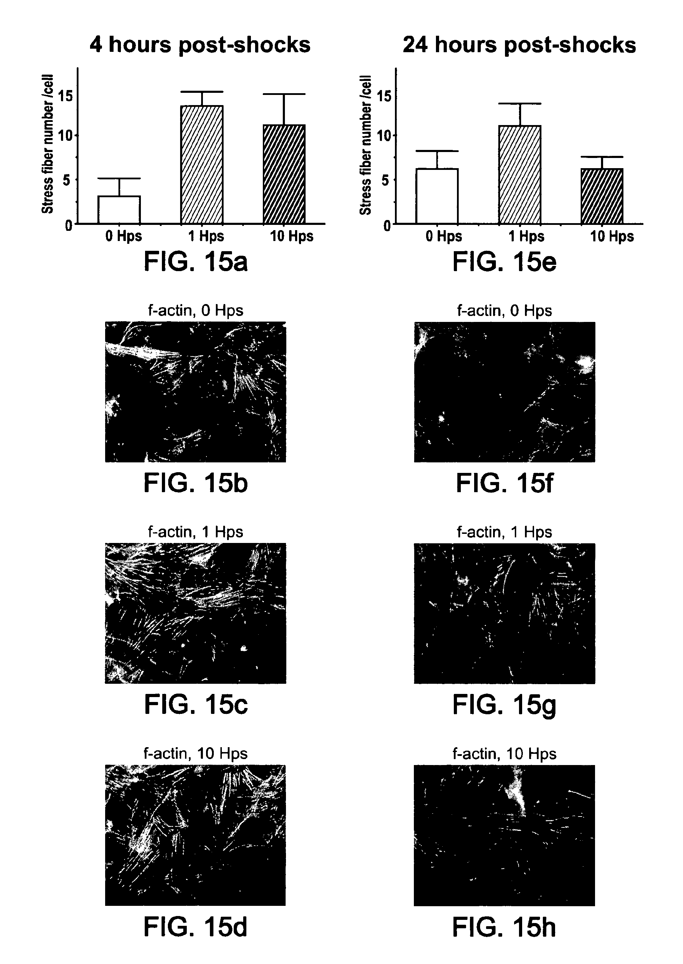

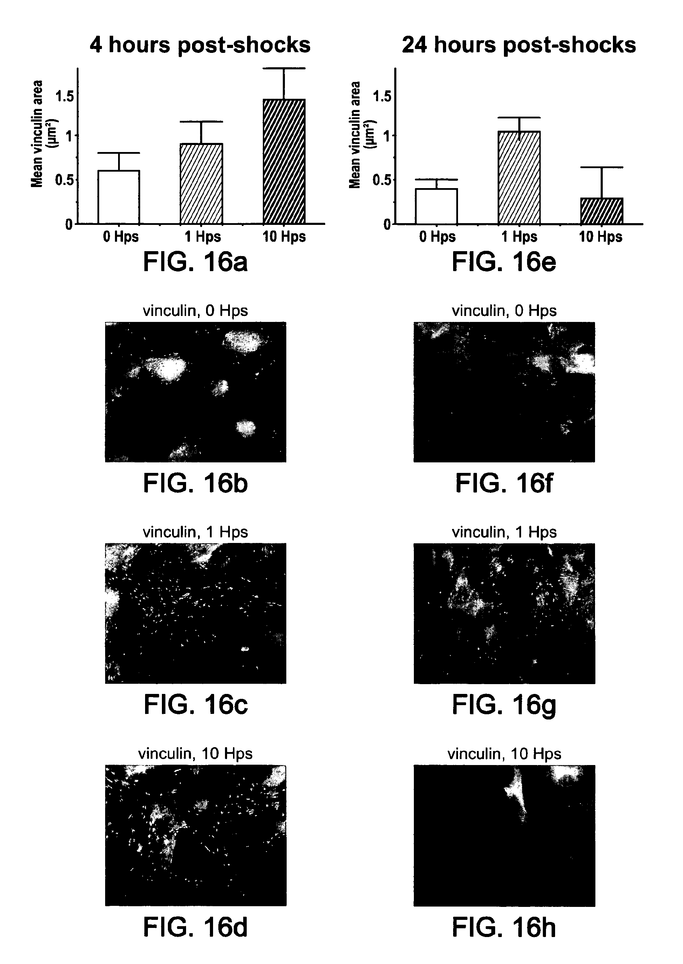

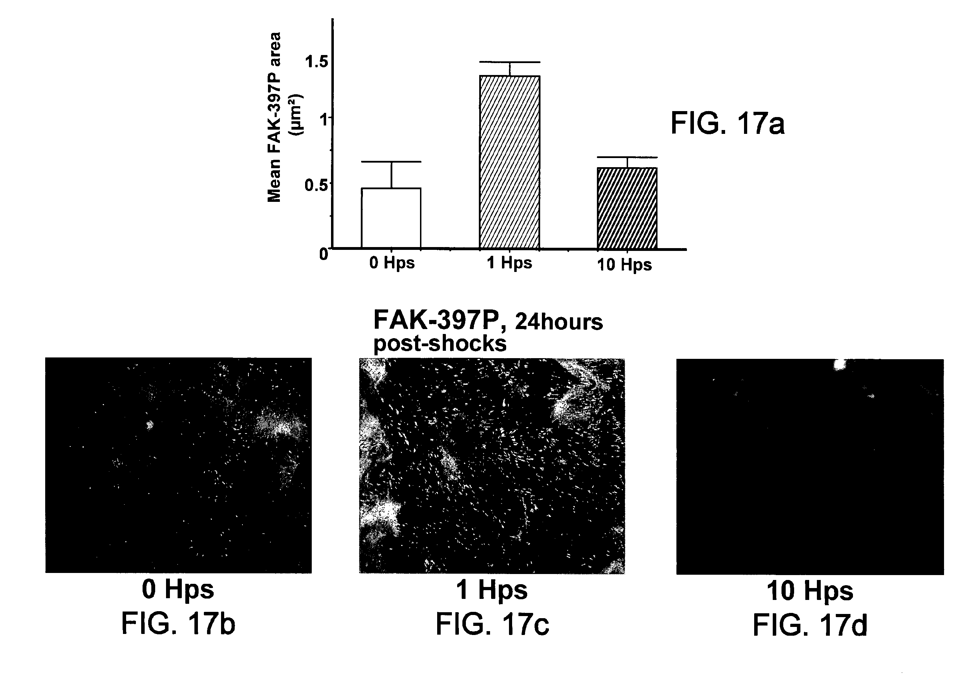

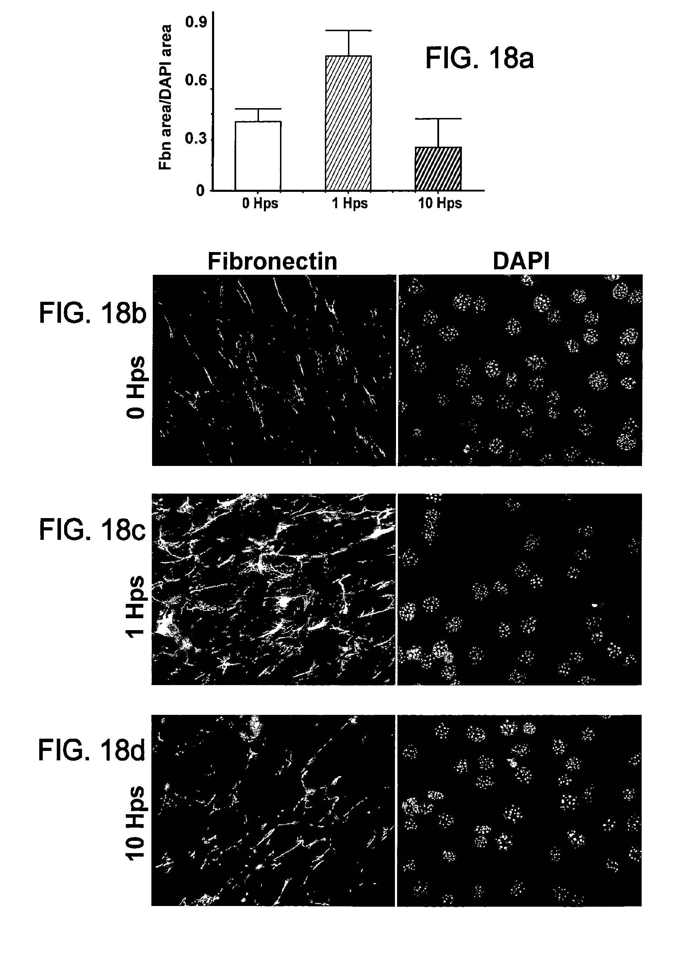

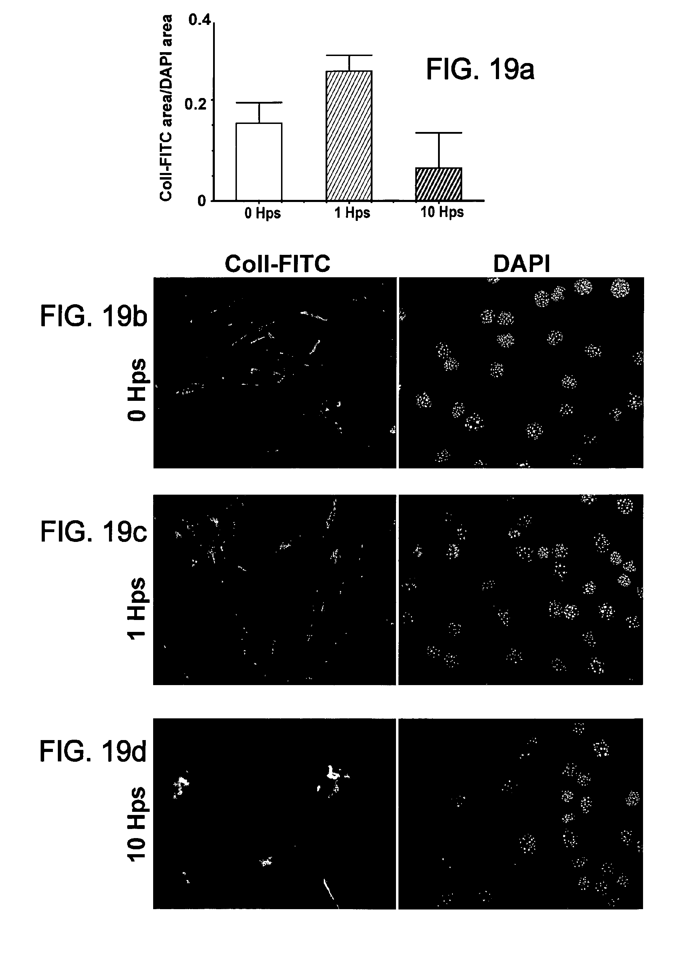

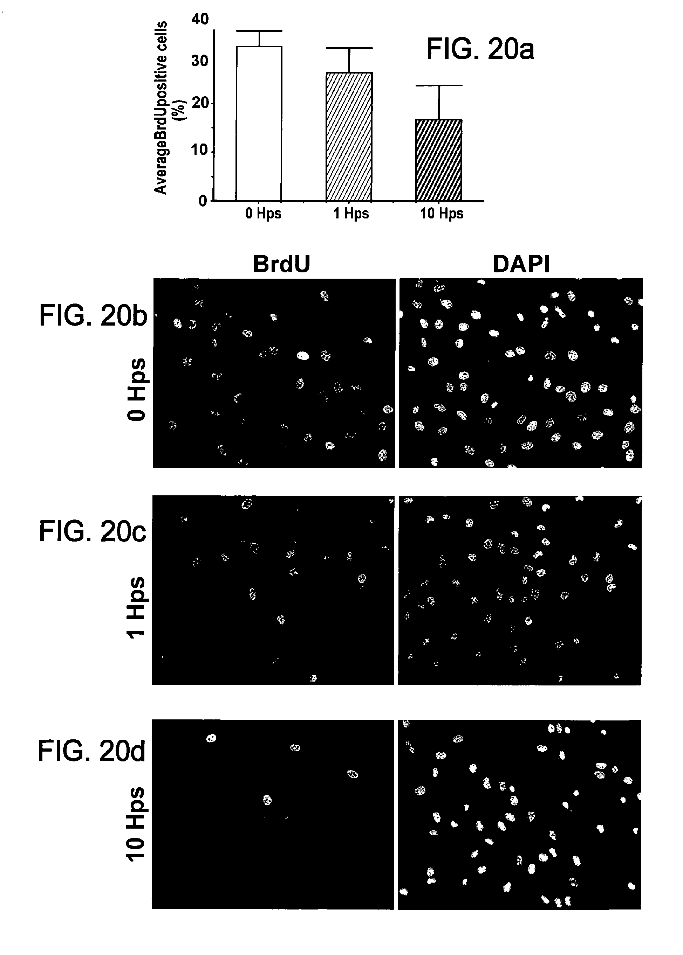

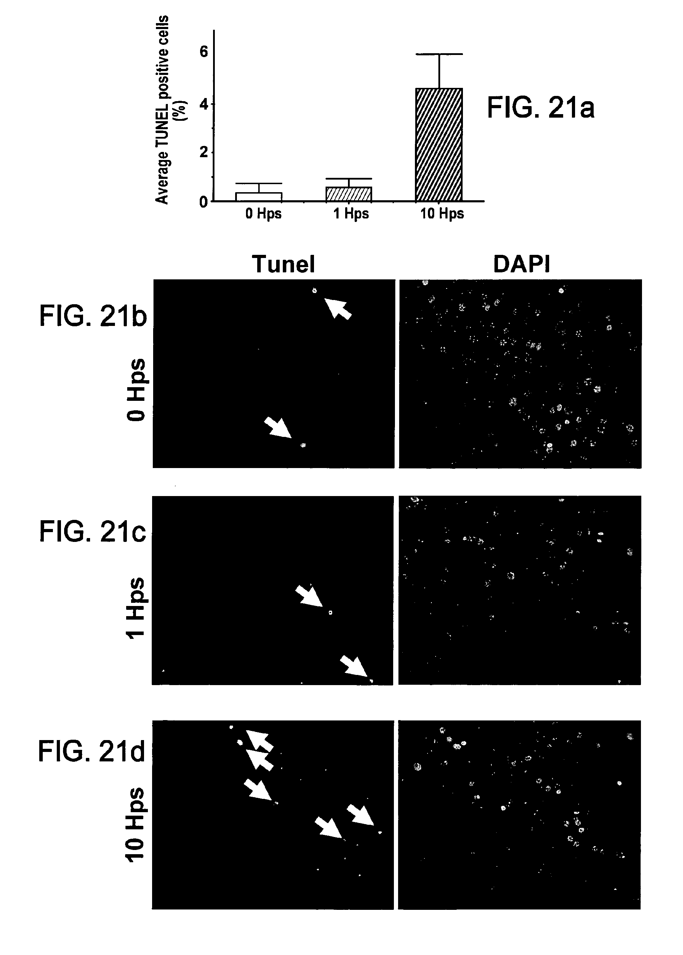

Depending on fluorescence staining, various approaches are used:
- for F-actin (Figures 15a-15h), raw fluorescence images are treated to remove the background (rolling ball radius: 10); cells are manually delineated (ROI), an automatic threshold is applied and binary images are skeletonised to extract the number of fibrillar stress fibers per ROI,
- for vinculin (Figures 16a-16h) and FAK-P (Figures 17a-17d), cells are manually delineated; the binary masks of spots were obtained by means of a dark top-hat transform; the dark top-hat is the same operation as the bright top-hat but with a preliminary inversion of the contrast of images (i.e. negative image); morphological features such as cell area, number of spots, mean area of spot per cell, are calculated by the image analysis program,
- for fibronectine (Figures 18a-18d) and collagen-FITC (Figures 19a-19d), the approach described for vinculin is used except that it is not possible to appraised cell area, so it has been decided to normalize the fluorescent area calculated by field (or image) by the DAPI area of the respective field as indicative of the number of cells per image,
- for BrdU (Figures 20a-20d) and Annexin-V (Figures 21a-21d) staining, the approach described for vinculin is used too, a morphological closing operation is applied on binary images to connect small objects and the number of spots (fluorescent nuclei) is calculated by field, this number is divided by the total number of cells calculated on the corresponding DAPI image. The average percentage of BrdU and tunnel positive cells is plotted.

### Cells Proliferation and Apoptosis

Cell proliferation is measured using a 5-Bromo-2'-deoxy-uridine Labeling and Detection Kit I (Roche Diagnostic, Meylan, France). After mechanical stimulation, cells are incubated during forty-eight hours to allow BrdU incorporation. After fixation, cells are imaged and quantification of average percentage of proliferating cells is performed.

Cell death is determined by using an Annexin-V-FLUOS Staining Kit purchased from Roche Diagnostic, Meylan, France. This kit enables the detection of apoptotic and necrotic cells. After fixation, twenty-four hours post-shocks, cells are imaged and quantification of average percentage of apoptotic cells is performed.

### Data analysis and statistics

For signal acquisition experiments, shocks tests are repeated 10 times using 10 different discs. Statistical analyses are performed with Mann-Whitney non-parametric test for unpaired samples. Statistical significance is assumed when the p-value is 0.05 or lower. For in vitro experiments with MC3T3-E1 cells, all data are obtained from at least two independent experiments performed in triplicate. One way analysis of variance (ANOVA) is performed. When F values for a given variable were found to be significant, the sequentially rejecting Bonferroni-Holm test is subsequently performed using the Holm's adjusted p-values from the t table.

### Results

### Mechanical shocks characterisation

In order to precisely define the mechanical information received by cell seeded on the bearing surface 2 of the titanium disc, the signal output by the accelerometer 50 fixed on the upper face 1a of disc has been analysed according to the force applied output by the force sensor 51 on the contact area with a square type signal (0V/+10V). Shocks parameters were designed to encompass energy of 160 pJ/µm², during 500 cycles (total energy of 75 nJ/µm²) between 1 and 10 Hps.

From the acceleration signal recorded, the kinetic energy of the Ti6Al4V disc 1 - accelerometer 50 unit during the mechanical shock is computed with the classical formula *Ek* = 1/2*mv* (*t*)² dt. (*Ek*: kinetic energy, *m*: mass, *v*: speed, given for the Ti6A14V disc - accelerometer unit).

For a shock of 35 N (Figure 12), the acceleration signal recorded reached 783±32g (Figure 13). Using these parameters, the kinetic energy calculated per surface unit was equal to 4.8±0.1 pJ/µm² per hit.

In the force signal (Figure 12), four rebounds can be observed, characterized by four peaks in the acceleration signal (Figure 13). In our calibrated system, 35 N shocks were delivered at 1 and 10 Hps for 30 minutes transmitting to cells total energies of ∼9 and ~90 nJ/µm² respectively.

On Figure 14, a vibratory analysis of the support using the acceleration signal enables to determine a frequency signature of the support 1.

### Effects of shocks on activation of focal adhesion contacts

Figures 16b, 16c, 16d, 16f, 16g and 16h present, on double stained cells for vinculin and phalloidin, effects of shocks delivered at 1 and 10 Hps compared to static control.

Four hours post-shocks, vinculin staining indicates that focal adhesion contacts were organized in larger clusters under shocks (1 and 10 Hps) (Figures 16c, 16d) as compared to control (diffuse pattern on Figure 16b). The mean vinculin area was increasing significantly from 0.75±0.2 µm² in control to 1±0.3 µm² at 1 Hps and up to 1.5±0.4 µm² at 10 Hps (Figure 16a).

In line with clustering of focal adhesion, stress fibers formation (F-actin polymerisation) was increased four hours post-shocks whatever the conditions (Figures 15c, 15d) as compared to control (Figure 15b). The number of stress fibers per cell increased significantly from an average of 4±2 in control to 14±3 at 1 and 12±4 at 10 Hps (Figure 15a).

Twenty-four hours post-shocks, vinculin staining is mainly diffused (Figures 16f, 16h) except under shocks at 1 Hps where focal adhesion contacts were still organized in larger clusters (Figure 16g). The mean vinculin area is increasing significantly from 0.5±0.1 µm² in control to 1.2±0.2 µm² at 1 Hps and decreased down to 0.3±0.4 µm² at 10 Hps (Figure 16e).

In line with clustering of focal adhesion at 1 Hps, stress fibers formation is maintained twenty-four hours post-shocks only at 1Hps (Figure 15g) as compared to control (Figure 15f) and 10 Hps (Figure 15h). The number of stress fibers per cell increased significantly from an average of 7±2 in control and 7±1 at 10 Hps to 12±3 at 1 Hps (Figure 15e). In contrast with results obtained four hours post-shocks, shocks delivered at 10 Hps was not able to maintain stress fibers and large focal adhesion contacts twenty-four hours post-shocks.

As for focal contacts activity, the phosphospecific FAK-397P immunostaining twenty-four hours post-shocks (Figures 17a-17c) has been quantified.

In control cells (Figure 17b), as well as for 10 Hps condition (Figure 17d), FAK-397P active contacts were represented by small spots of fluorescence, in contrast for 1 Hps condition where larger area of spots indicate a sustained activity of focal contacts (Figure 17c); quantitative data clearly indicate that active contacts were larger at 1 Hps as compared to control and 10 Hps (Figure 17a). These data corroborated those obtained on vinculin and F-actin organisation and indicated that shocks delivered at 1 Hps are particularly efficient as focal contact activator.

### Effects of shocks on fibrillogenesis

As sustained phosphorylation of FAK can be explained by the secretion of matrix protein and that FAK-P is involved in fibrillogenesis, the production of fibronectin (Fbn) has been tested by immunofluorescence as well as fibrillogenesis of soluble fluorescent type I collagen added after shocks.

Figures 18b, 18c and 18d present fibronectine staining with its respective DAPI staining in order to normalize the fibronectine area by the DAPI area.

Fibronectin staining revealed an oriented fibrillar organization in thin fibers for control cells (Figure 18b) and thicker for 1 Hps (Figure 18c) and for 10 Hps (Figure 18d) forty-eight hours post-shocks. It can be observed that staining at 10 Hps was not homogeneously distributed. Quantification analysis indicated that the relative fibronectin area at 1 Hps (0.7±0.2) was significantly enhanced as compared to control (0.4±0.1) and 10 Hps (0.25±0.2) (Figure 18a).

To confirm that fibrillogenesis was increased by shocks delivered at 1 Hps, soluble collagen (Coll-FITC) has been incubated immediately at the end of shocks to test the ability of cells to incorporate this exogenous collagen for forty-eight hours.

Coll-FITC images demonstrated that control cells (Figure 19b) and cell stimulated at 1 Hps (Figure 19c) were able to organize fibers of collagen as compared to the 10 Hps condition where fluorescent collagen was mainly organised in spots and not into fibers (Figure 19d). Quantitative analysis of relative area of Coll-FITC demonstrated that shocks delivered at 1 Hps significantly increased fibrillogenesis (0.3±0.05) as compared to control (0.18±0.02) and 10 Hps (0.07±0.08) (Figure 19a).

### Effects of shocks on Cells proliferation and apoptosis

In order to confirm the putative deleterious effect (reduced focal contact maturation and fibrillogenesis) observed at 10 Hps, BrdU incorporation during forty-eight hours post-shocks has been quantified.

Figures 20b, 20c, 20d, 21b, 21c and 21e represent nuclei double stained for BrdU, indicative of proliferative cells, or Annexin-V indicative of apoptotic or necrotic cells with their corresponding DAPI staining, indicating the total number of cells per field.

Figure 20d showed that BrdU positive cell percentage was reduced as compared to images of static control (Figure 20b). Quantification of data demonstrated that shocks delivered at 10 Hps reduced significantly proliferation capability of strained cells, decreasing from 35±5% of BrdU positive cells for control and 30±8% at 1 Hps down to 19±9% (Figure 20a).

Furthermore, it has been demonstrated with Annexin-V staining that Annexin-V positive cells (indicated by arrows) are more numerous in the 10 Hps condition (Figure 21d) as compared to other conditions where positive cells were almost absent (Figures 21b, 21c). Quantitative data demonstrated that shocks delivered at 10 Hps induce a little but significant increase in Annexin-V staining. Percentage of dead cells increases from 1.2±0.6% for control and 1.5±0.5% at 1 Hps up to 5±1% at 10 Hps (Figure 21a).

Thus, the method and apparatus of the invention have led to adjust mechanical shocks or impacts that stimulate bone reparation or osteoblastic activity.

The signal acquisition showed that the frequency content due to the shock can be very high (35 kHz) despite the low applying frequencies. Different energies were delivered to the cells during impacts and vibrations of the support 1 may influence osteoblasts which are in contact with the support 1.

## Claims

1. A method for applying a mechanical loading to cells, comprising:
- a step of providing a support (1) having a bearing surface (2) adapted to receive said cells,
- a step of providing cells on the bearing surface (2) of said support (1),
- a step of placing said support (1) provided with the cells within a chamber (4) adapted to house said support (1),
- at least one step of applying a mechanical loading to the support (1) by a loading device (5) which extends at least in part in the chamber (4),
said method being **characterised in that**, at the step of applying the mechanical loading, a dynamic loading in vibration is applied to the support (1), said dynamic loading having a magnitude and a frequency and being adapted to cause the support (1) to vibrate.

2. The method according to claim 1, wherein the step of applying the dynamic loading comprises applying an impact.

3. The method according to claim 1 or 2, wherein the step of applying the dynamic loading comprises varying said magnitude in a range of magnitudes and/or said frequency in a range of frequencies.

4. The method according to any of claims 1 to 3, wherein the dynamic loading is applied perpendicularly to the bearing surface (2).

5. The method according to any of claims 1 to 4, wherein the dynamic loading is applied parallel to the bearing surface (2).

6. The method according to any of claims 1 to 5, wherein the dynamic loading is applied to a surface that represents more than 50% of a plane face (1b) of the support (1) which faces the loading device (5).

7. The method according to any of claims 1 to 6, being implemented for cell culture under dynamic loading.

8. The method according to claim 7, further comprising, after the step of providing cells on the bearing surface (2) of said support (1), placing the support (1) in a culture environment.

9. The method according to claim 8, wherein the chamber (4) is adapted to be closed in a tight way, the culture environment comprises a fluid culture medium, said placing the support (1) in the culture environment comprising, after the step of placing said support (1) within the chamber (4), closing the chamber (4) in a tight way and supplying the chamber (4) with said fluid culture medium.

10. The method according to claim 8 or 9, wherein said placing the support (1) in the culture environment comprises placing the support (1) in an incubator.

11. The method according to any of claims 7 to 10, wherein the cells are adherent cells, the support (1) is a substrate made of a biomaterial.

12. The method according to claim 11, being implemented for making an implant.

13. The method according to any of claims 1 to 12, further comprising a step of determining dynamic properties of the support (1) by a dynamic data collection device.

14. The method according to claim 13, wherein the step of determining the dynamic properties of the support (1) comprises measuring, recording and processing acceleration of the support (1).

15. The method according to claim 14, wherein the step of determining the dynamic properties of the support (1) comprises performing a vibratory analysis of the support (1).

16. The method according to any of claims 13 to 15, wherein the step of determining the dynamic properties of the support (1) comprises measuring, recording and processing force applied to the support (1).

17. The method according to any of claims 13 to 16, wherein the step of applying the dynamic loading comprises controlling the magnitude and/or the frequency of dynamic loading according to the determined dynamic properties of the support (1).

18. The method according to any of claims 1 to 17, further comprising, after the step of applying the dynamic loading, a step of analysing cell response to dynamic loading.

19. Apparatus for implementing the method according to any of claims 1 to 18, comprising:
- a frame (3),
- a chamber (4) mounted on the frame (3), adapted to house a support (1), said support (1) having a bearing surface (2) adapted to receive cells,
- a loading device (5) mounted on the frame(3), extending at least in part in the chamber (4) and adapted to apply a mechanical loading to the support (1),
said apparatus being **characterised in that** the loading device is adapted to apply to the support (1) a dynamic loading in vibration, said dynamic loading having a magnitude and a frequency and being adapted to cause the support (1) to vibrate.

20. The apparatus according to claim 19, wherein the loading device (5) is adapted to apply an impact.

21. The apparatus according to claim 19 or 20, wherein the loading device (5) is adapted to vary said magnitude in a range of magnitudes and/or said frequency in a range of frequencies.

22. The apparatus according to any of claims 19 to 21, wherein the loading device (5) is adapted to apply the dynamic loading perpendicularly to the bearing surface (2).

23. The apparatus according to any of claims 19 to 22, wherein the loading device (5) is adapted to apply the dynamic loading parallel to the bearing surface (2).

24. The apparatus according to any of claims 19 to 23, wherein the support (1) has a plane face (1b) which faces the loading device (5), the loading device (5) being adapted to apply the dynamic loading to a surface that represents more than 50% of said face (1b).

25. The apparatus according to any of claims 19 to 24, wherein the chamber (4) is adapted to be closed in a tight way and is provided with a supply device for a fluid culture medium.

26. The apparatus according to any of claims 19 to 25, further comprising a dynamic data collection device adapted to determine dynamic properties of the support (1).

27. The apparatus according to claim 26, wherein the dynamic data collection device comprises at least an acceleration sensor (50) adapted to output a signal representing acceleration of the support (1), and a central unit, which acceleration sensor (50) is connected to, said acceleration sensor (50) being adapted to measure the acceleration of the support (1), said central unit being adapted to record and process the signal output by the acceleration sensor (50).

28. The apparatus according to claim 27, wherein the central unit is adapted to perform a vibratory analysis of the support (1).

29. The apparatus according to claim 27 or 28, wherein the dynamic data collection device further comprises at least a force sensor (51) adapted to output a signal representing force applied to the support (1), said force sensor (1) being connected to the central unit and being adapted to measure the force applied to the support (1), said central unit being adapted to record and process the signal output by the force sensor (51).
